# EUROPÄISCHE PATENTSCHRIFT

(11) **EP 1 517 988 B1**
(45) Veröffentlichungstag und Bekanntmachung des Hinweises auf die Patenterteilung: **08.05.2013**
(21) Anmeldenummer: 03760661.3
(22) Anmeldetag: 20.06.2003
(51) Int. Cl.: A61K 38/18

(54) **Verwendung von Erythropoietin zur Geweberegeneration in vivo**
Use of erythropoietin for tissue regeneration in vivo
Utilisation de l'érythropoïétine pour la régénération de tissus in vivo

(30) Priorität: 20.06.2002 DE 10227611; 26.07.2002 DE 10234204
(43) Veröffentlichungstag der Anmeldung: 30.03.2005
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen
(86) Internationale Anmeldenummer: PCT/EP2003/006509
(87) Internationale Veröffentlichungsnummer: WO 2004/001023

(56) Entgegenhaltungen:
- EP-A- 0 358 506
- WO-A-01/48153
- WO-A-90/10647
- WO-A-96/27657
- WO-A2-00/34443
- US-A- 4 963 489
- US-A- 5 919 702
- US-B1- 6 319 942
- RATAJCZAK J ET AL: "IMPROVED SERUM FREE SYSTEM FOR CLONING HUMAN PURE ERYTHROID COLONIES. THE ROLE OF DIFFERENT GROWTH FACTORS AND CYTOKINES ON BFU-E FORMATION BY THE BONE MARROW AND CORD BLOOD CD34+ CELLS" FOLIA HISTOCHEMICA ET CYTOBIOLOGICA, VESALIUS UNIVERSITY MEDICAL PUBLISHER, KRAKOW,, PL, Bd. 36, Nr. 2, 1998, Seiten 55-60, XP000886615 ISSN: 0239-8508
- KAWAI K ET AL: "Accelerated tissue regeneration through incorporation of basic fibroblast growth factor-impregnated gelatin microspheres into artificial dermis - targeted delivery systems" BIOMATERIALS, ELSEVIER SCIENCE PUBLISHERS BV., BARKING, GB, Bd. 21, Nr. 5, März 2000 (2000-03), Seiten 489-499, XP004185546 ISSN: 0142-9612
- CARDIER ET AL: "THROMBOPOIETIN AND ITS RECEPTOR, C-MPL, ARE CONSTITUTIVELY EXPRESSED BY MOUSE LIVER ENDOTHELIAL CELLS", BLOOD, vol. 91, no. 3, 1 February 1998 (1998-02-01), pages 923-929,
- ISHII ET AL: "HEPATOCYTE GROWTH FACTOR STIMULATES LIVER REGENERATION AND ELEVATES BLOOD PROTEIN LEVEL IN NORMAL AND PARTIALLY HEPATECTOMIZED RATS", JOURNAL OF BIOCHEMISTRY, vol. 117, no. 5, May 1995 (1995-05), pages 1105-1112,
- KLEMM K ET AL: 'ERYTHROPOIETIN VERBESSERT DIE LEBERREGENERATION DURCH STIMULATION DER ZELLPROLIFERATION UND INHIBITION DER ZELLAPOPTOSE', [Online] 02 Mai 2006, Gefunden im Internet: <URL:http://www.egms.de/en/meetings/dgch200 6/06dgch148.shtml>
- LABAHN R: 'HAUTREGENERATION MIT ERYTHROPOIETIN BEI VERBRENNUNGEN', [Online] 12 Juni 2007, Gefunden im Internet: <URL:http://idw-online.de/pages/de/news?pri nt=1&id=213191>
- BAGNIS C ET AL: "ERYTHROPOIETIN ENHANCES RECOVERY AFTER CISPLATIN-INDUCED ACUTE RENAL FAILURE IN THE RAT", NEPHROLOGY, DIALYSIS, TRANSPLANTATION, vol. 16, 2001, pages 932-938,
- BUEMI ET AL: "RECOMBINANT HUMAN ERYTROPOIETIN (RHUEPO): MORE THAN JUST THE CORRECTION OF UREMIC ANEMIA", JOURNAL OF NEPHROLOGY, vol. 15, 2002, pages 97-103,
- JUUL S E: "ERYTHROPOIETIN IN THE NEONATE", CURRENT PROBLEMS IN PEDIATRICS, MOSBY, 1 May 1999 (1999-05-01), pages 133-149, XP001167109, ISSN: 0045-9380, DOI: 10.1016/S0045-9380(99)80055-3
- MACHENS: "WACHSTUMSFAKTOREN DER ANGIOGENESE", MEDREPORT, vol. 16, 2008,
- CURRIE L J ET AL: "The use of fibrin glue in skin grafts and tissue-engineered skin replacements: a review", PLASTIC AND RECONSTRUCTIVE SURGERY, WILLIAMS AND WILKINS CO., BALTIMORE, MD, US, vol. 108, no. 6, 1 November 2001 (2001-11-01), pages 1713-1726, XP002973214, ISSN: 0032-1052, DOI: 10.1097/00006534-200111000-00045
- BUEMI M ET AL: "Recombinant Human Erythropoietin Influences Revascularization and Healing in a Rat Model of Random Ischaemic Flaps", ACTA DERMATO-VENEREOLOGICA, TAYLOR & FRANCIS LTD, UNITED KINGDOM, vol. 82, no. 6, 1 January 2002 (2002-01-01), pages 411-417, XP002993404, ISSN: 0001-5555, DOI: 10.1080/000155502762064520
- MULDER G: "DIABETIC FOOT ULCERS", NEPHROLOGY, DIALYSIS, TRANSPLANTATION, vol. 16, 2001, pages 695-698, XP001091116,
- "ARZNEIMITTELRICHTLINIEN: BECAPLERMIN", BUNDESANZEIGER, vol. 137, July 2000 (2000-07), pages 1-3, XP001091115,
- LAUER G ET AL: "EXPRESSION AND PROTEOLYSIS OF VASCULAR ENDOTHELIAL GROWTH FACTOR IS INCREASED IN CHRONIC WOUNDS", JOURNAL OF INVESTIGATIVE DERMATOLOGY, vol. 115, 2000, pages 12-17,
- WANG FF ET AL: "SOME CHEMICAL PROPERTIES OF HUMAN ERYTHROPOIETIN", ENDOCRINOLOGY, vol. 116, 1985, pages 2286-2292,
- OGILVIE M ET AL: "ERYNTHROPOIETIN STIMULATES PROLIFERATION AND INTERFERES WITH DIFFERENTIATION OF MYOBLASTS", JOURNAL OF BIOLOGICAL CHEMISTRY, THE AMERICAN SOCIETY OF BIOLOGICAL CHEMISTS, INC, US, vol. 275, no. 50, 1 January 2000 (2000-01-01), pages 39754-39761, XP001167111, ISSN: 0021-9258, DOI: 10.1074/JBC.M004999200 [retrieved on 2000-09-19]
- XURI L ET AL: "PDGF-C IS A NEW PROTEASE-ACTIVATED LIGAND FOR THE PDGF .ALPHA.-RECEPTOR", NATURE CELL BIOLOGY, vol. 2, 2000, pages 302-309,
- BERGSTEN E ET AL: "PDGF-D IS A SPECIFIC, PROTEASE-ACTIVATED LIGAND FOR THE PDGF .BETA.-RECEPTOR", NATURE CELL BIOLOGY, vol. 3, 2001, pages 512-516,

## Beschreibung

Die vorliegende Erfindung betrifft die Verwendung von Erythropoietin (EPO) in isolierter Form zur Regeneration von Geweben, insbesondere die Haut.

In der Ontogenese, also der Keimesentwicklung des Einzelwesens, werden Wachstumsfaktoren exprimiert, die grundlegende strukturelle und hinsichtlich der Zellzahl numerische Prozesse auslösen können. Im wachsenden Organismus geht die Fähigkeit zu strukturellen Reparaturen durch Regeneration zunehmend verloren, da diese Wachstumsfaktoren nicht mehr exprimiert werden. Faktoren des Knochenmarks bzw. der blutbildenden Organe sind während spezifischen ontogenetischen Prozessen an Wachstumsprozesse anderer Organe zeitlich gekoppelt.

Ein Nachteil bekannter Wachstumsfaktoren, wie z.B. "Epidermal Growth Factor" (EGF), "Vascular Endothelial Growth Factor" (VEGF) oder "Hepatocyte Growth Factor" (HGF), ist dass die Vermehrungspmzesse, insbesondere beim Einsatz primärer Zellen in vitro limitiert sind und dass der Einsatz in vivo wegen möglicher Nebenwirkungen, wie z.B. der Aktivierung von Onkogenen , problematisch ist.

Die genaue Kenntnis der Faktoren und deren kontrollierten Gabe wäre daher sowohl ein wichtiger Faktor, um Zellen, insbesondere im Bereich des Tissue Engineering, entsprechend vermehren und differenzieren zu können als auch um strukturelle Prozesse der dreidimensionalen (3-D) Regeneration zu induzieren.

Insbesondere beim Tissue Engineering stehen derartige strukturelle Vorgaben im Vordergrund, wobei das 3-D-Wachstum und dessen Initiation bisher nicht verstanden ist. Konventionelle Ansätze wie Aggregatkulturen erreichen zwar eine hohe Dichte, müssen aber mit vorexpandierten oder aus primären Geweben isolierten Zellen, z. B. Hepatozyten, aufgebaut werden. Ein induktiver Wachstumsprozess in eine bestimmte, vorgegebene Struktur war bisher nicht möglich.

Ein formschaffendes Wachstum aus wenigen Vorläuferzellen in eine 3-D-Struktur und ein induktives Verhalten für Nachbarschaftsprozesse im Sinne einer Geweberegeneration in vitro und in vivo war bisher nicht möglich. Ein induktive Wachstumsverhalten von Zellen bedeutet jedoch gerade für therapeutische oder biotechnologische Prozesse eine erhebliche Innovation. Ein derartiges Wachstumsverhalten sollte, unterstützt durch eine 3D Trägermatrix, ein Wachstum nicht nur im Sinne einer Besiedlung oder einem strukturellen Remodelling erlauben, sondern tatsächlich eine gerichtete de novo Formierung aus einem Induktionsnukleus erlauben können. Derartige Prozesse laufen in der Ontogenese ab und bauen auf eine vorher existierende Anlage auf.

Bekannt ist lediglich, dass Wachstumsfaktoren, insbesondere bei neuronalen Progenitoren fötalen Ursprungs wie z.B. "Leukemia Inhibitory Factor" (LIF), "Ciliary Neurotropic Factor" (CNTF), "Glial Derived Neurotrophic Factor" (GDNF) oder "nerve growth factor" (NGF), eine Proliferationsphase undifferenzierter Neuronen ermöglichen. Nach Erreichen einer Differenzierung können diese Faktoren jedoch nicht mehr wirken. Buemi et al., J. Nephrol. 2002; 15: 97-103, beschreiben, dass EPO eine wichtige Rolle bei der Neovaskularisierung und Wundheilung Zukammt.

Es ist daher wünschenswert, ein Regenerationsverfahren in vivo zu schaffen, das den physiologischen Zustand dieser Zellsysteme im wesentlichen erhalten kann und ein im wesentlichen strukturelles Wachstum ermöglicht.

Es wurde nun gefunden, dass die Verwendung des Wachstumsfaktors Erythropoietin (EPO) die Vermehrung und Differenzierung von Zellen einleitet bzw. terminiert und strukturell lenkt insbesondere bei der Gewebe regeneration in vivo, vorzugsweise bei Haut.

Überraschenderweise wurde hierdurch nicht nur eine Vermehrung der Zellen, sondern auch eine Induzierung struktureller Prozesse bewirkt, insbesondere wenn über eine induktive Wirkung bei einem Implantat vor Ort (in situ) beispielsweise über einen sogenannten Homingprozess eine lokal spezifische Zellvermehrung und gerichtete Differenzierung bewirkt wird. Das bedeutet, dass die Wachstumshormone diese strukturellen Prozesse auslösen aber auch terminieren können.

Wachstumsfaktoren sind im allgemeinen käuflich zu erwerben, können aber auch gentechnisch nach dem Fachmann bekannten Methoden hergestellt werden. Sie umfassen nicht nur die natürlich vorkommenden Wachstumsfaktoren, sondern auch Derivate oder Varianten mit im wesentlichen derselben biologischen Aktivität.

EPO wird auch als embryonale Form des TPO bezeichnet und ist mit seinen Varianten z. B. in der EP 0 148 605, EP 0 205 564, EP 0 209 539, EP 0 267 678 oder EP 0 411 678 beschrieben.

Der Begriff Wachstumsfaktor ist folglich gemäß der vorliegenden Erfindung nicht nur auf die natürlich vorkommenden Formen beschränkt, sondern umfasst auch nicht natürlich vorkommende Formen bzw. Varianten oder Derivate. Der Begriff Wachstumsfaktor umfasst gemäß der vorliegenden Erfindung nicht nur Wachstumsförderer, sondern auch Wachstumsinhibitoren, wie z. B. Somatostatin, TGF beta und/oder Prostaglandine. Derartige Wachstumsinhibitoren eignen sich insbesondere zur Unterdrückung bzw. Hemmung des Wachstums von veränderten Zellen, wie z. B. Tumorzellen, indem diese gleichzeitig oder sequenziell beispielsweise auch mittels Hydrogele oder Slow-release Materialien hochkonzentriert, lokal eingesetzt werden.

Das erfindungsgemäße Verfahren kann zur lokalen Applikation in vivo eingesetzt werden, in dem Wachstumsfaktoren entweder allein oder in Kombination als Mischung oder sequenziell eingesetzt werden oder in Kombination mit den genannten biologischen Matrizes oder Trägerstrukturen, beispielsweise für die Geweberegeneration, wie z. B. die Leberregeneration, Herzmuskelregeneration oder für die Wundheilung Bereich.Haut, z.B. bei diabetischen Ulzera, oder Gingiva. Beispielsweise können sie in einem Hydrogel, z. B. Fibrin und/oder ein Polymere, wie z. B. Polylaktid oder Polyhydroxyalkanoat, und/oder ein Alginat, auf die Resektionsfläche z. B. einer Leber zur Leberregeneration aufgebracht werden oder bei z. B. akutem Leberversagen über einen Port mit Hilfe eines Katheters lokal oder systemisch appliziert werden.

Folglich bezieht sich die vorliegende Erfindung auch auf die Verwendung von EPO zur Herstellung eines Arzneimittels zur Behandlung der Regeneration von Gewebe, insbesondere Haut, zur Unterstützung des Wundheilungsprozesses, insbesondere im Bereich Haut, vorzugsweise bei diabetischer Ulzera oder Gingiva und/oder zur Verbesserung der Wundheilung und Geweberegeneration z.B. nach Operationen, zur Anregung der Gefäßneubildung und Regeneration und/oder Ischämien nach Verletzungen und Trauma und/oder Regeneration von Geweben im Anschluss an eine Gewebeverletzung.

Als Wachstumsfaktor können zusätzlich "Transforming Growth Factor beta" (TGF beta), Prostaglandine, Granulozyten-Makrophagen-stimulierender Faktor (GM-CSF), "Growth Hormone Releasing Hormone" (GHRH), "Thyrotropin-releasing Homone" (TRH), "Gonadotropinreleasing Hormone" (GnRH), "Corticotropin-releasing Hormone" (CRH), Dopamin, "Antidiuretic Hormon" (ADH), Oxytocin, Prolactin, Adrenocorticotropin, beta-Celltropin, Lutrotropin und/oder Vasopressin verwend werden bzw. zusätzlich ein oder mehrere Nervenregenerationsfaktoren, vorzugsweise "nerve growth factor" (NGF) und/oder ein oder mehrere Gefäßregenerationsfaktoren, vorzugsweise "Vascular Endothelial Growth Factor" (VEGF) und/oder "Plateled Derived Growth Factor" (PDGF).

Die in der vorliegenden Erfindung dargestellten weiteren Ausgestaltungsformen gelten in analoger Weise auch für die beschriebenen erfindungsgemäßen Verwendungen.

Des weiteren kann eine biologische Matrix oder Trägerstruktur enthaltend EPO, als induktives Substrat für 3-D Wachstum und/oder Regeneration innerhalb einer Vermehrungsphase oder nach einer Vermehrungsphase zur Differenzierung oder zum Wachstumsarrest benutzt werden.

Die biologische Matrix oder Trägerstruktur stellt beispielsweise ein Implantat, ein Transplantat und/oder ein Trägermaterial zum Wachstum von Zellen dar, wobei die biologische Matrix oder Trägerstruktur ein Stent, ein Katheter, eine Haut, ein Hydrogel, ein Knochenersatzmaterial, ein allogenes, autologes oder xenogenes, azellularisiertes oder nicht-azellularisiertes Gewebe, ein synthetisches Gewebe, ein Feeder-Layer oder ein Fließ, wie z. B. ein Fließ aus Collagen, Laminin und/oder Fibronektin mit oder ohne synthetischer oder anderweitiger Grundstruktur, wie z. B. Kunststoff oder eine biologische Matrix, sein kann. Beispielhafte Ausführungsformen sind bereits oben beschrieben.

Die biologische Matrix oder Trägerstruktur kann bereits mit gewebespezifischen Zellen, Vorläuferzellen, Knochenmarkszellen, peripheres Blut, Fettgewebe und/oder Fasergewebe vorbesiedelt oder für die in vivo Besiedelung bereits vorbereitet sein.

Die biologische Matrix oder Trägerstruktur kann auch mit einer (Bio)polymerschicht, die mindestens einen der genannten Wachstumsfaktoren enthält, beschichtet sein. Als (Bio)polymerschicht eignen sich z. B. Fibrin, Plasma, Collagen und/oder Polylaktide.

Die folgenden Beispiele sollen die Erfindung näher erläutern, ohne sie zu beschränken.

### Beispiele:

### Regeneration von Geweben in vivo

### a) Leber (nur zum Vergleich)

Nach Leberteilresektion wird dem Patient EPO systemisch und / oder topisch durch Aufbringen auf die Resektionsfläche in Verbindung mit einem Polymer verabreicht. Das Polymer kann ein Biopolymer sein wie z.B. Fibrin (aus z.B. Fibrinkleber), polymerisiertes Plasma, polymerisiertes Blut oder Bioklebsstoffe, z.B. Muschelkleber. Es kann sich aber auch um synthetische oder biologische Gele oder Hydrogele handeln. Auch kann das EPO in Fliesse, die der Blutungsstillung dienen, eingebracht werden (z.B. Kollagenfliesse, Tamponade, Gewebe und Gewirke).

Durch die Wirkung von EPO kommt es innerhalb von 2 Wochen zu einer Wiederherstellung des Originalvolumens der Leber. Hierbei kommt es nicht nur zu einer Vermehrung der Hepatozyten, sondern zu einem koordiniertem Wachstum bei dem auch die Gefäße, die Gallengänge wie auch die Kapselstrukturen bis zu ihrer Originalgröße zurückwachsen.

Bei 30 Tieren konnte gezeigt werden, dass die Regeneration der Leber signifikant gebenüber den Kontrolltieren (ohne EPO Gabe) erfolgte.

EPO kann zur Leberregeneration auch bei chronischen Lebererkrankungen wie z.B. Cirrhose, Fibrose, Hepatitis eingesetzt werden. Dadurch kann erstmals eine therapeutische Wirkung in Bezug auf das Leberparenchym erreicht werden.

### b) Entzündliche Darmerkrankungen (nur zum Vergleich)

Bei Patienten mit Morbus Crohn kommt es zu Wundheilungsstörungen im Bereich des Darmepithels. Auch darunterliegende Gewebestrukturen können im Rahmen von Entztindungsreaktionen in Mitleidenschaft gezogen sein. Bei diesen Patienten führt die systemische und / oder topische Gabe von EPO zu einer Wiederherstellung des Darmepithels durch Regeneration. Die topische Gabe kann über slow release Kapseln im Darmbereich oder durch Gabe von Zäpfchen mit Gelen oder lokalen Instillation mit Lösungen erfolgen.

Die Resorption in den regionalen Gefäßbereich kann durch die Gabe von pegylierten (PEG) Verbindungen optimiert werden, so dass über die regionale Gabe im Entzündungsbereich eine systemische Wirkung und damit die Initiation des Wundheilungsprozesses erfolgen kann.

Als prognostischer positiver Faktor bei Patienten mit Morbus Crohn ist das Vorliegen einer Anämie zu sehen. In der Vergangenheit wurde vermutet, dass die Anämie eine unabhängige Begleiterkrankung sei, bzw. auf die Auszehrung durch Resorptionsprobleme zurückzuführen sei. Unsere Ergebnisse zeigen, dass es sich bei der Wundheilungsstörung um einen Mangel an endogenem EPO handelt. Dadurch kann Morbus Crohn sehr selektiv durch eine exogene Gabe von EPO therapiert werden. Weitere Einsatzgebiete befinden sich auch im Bereich der Colitis Ulcerosa.

### c) Wundheilungsstörungen im Bereich der Haut

Bei Patienten bei diabetischen Ulzera bestehen trophische Störungen die einen Wundverschluss im Bereich meist der Beine erschweren. Die Fähigkeit zu einer strukturellen Geweberegeneration ist auf Grund der Basiserkrankung eingeschränkt. In diesen Fällen wird durch die topische Gabe von EPO eine Wundheilung induziert. Als vorteilhaft erweist sich die Applikation von EPO nach Aufrauhung des Untergrundes im Rahmen eines Debridements. Die Verbindung von EPO mit einer durch Calziumchlorid ausgelösten Polymerisation führt zur Integration von EPO in ein Blutkoagel, wodurch es zu einem topischen Slowreleasepräperat kommt. Alternativ kann EPO auch in Verbindung mit einem Fibrinkleber oder einem Fliess oder einer mit EPO imprägnierten Tamponade (z.B. Kollagenflies) verabreicht werden.

EPO kann in ähnlicher Weise bei allen anderen Wundheilungserfordernissen z.B. im Muskelbereich nach Sportverletzungen, Muskelerkrankungen, Knochenverletzungen, Weichteilverletzungen und generell zur Verbesserung der Wundheilung und Geweberegeneration z.B. nach Operationen, akuten und chronischen Erkrankungen gegeben werden.

## Patentansprüche

1. Verwendung von Erythropoietin (EPO) zur Herstellung eines Medikamentes zur topischen Behandlung der Haut bei der Wundheilung nach Aufrauhung des Untergrundes im Rahmen eines Debridements und Applikation von EPO, wobei das EPO durch Calciumchlorid ausgelöste Polymerisation in ein Blutkoagel integriert und aus diesem langsam lokal in die Wunde abgeben wird.

2. Verwendung von EPO zur Herstellung eines Medikamentes zur topischen Behandlung der Haut bei der Wundheilung nach Aufrauhung des Untergrundes im Rahmen eines Debridements und Applikation von EPO, wobei das EPO in Verbindung mit einem Fibrinkleber, einem Fliess oder einer mit EPO imprägnierten Tamponade verabreicht wird.

3. Verwendung nach Anspruch 1 oder 2 zur topischen Behandlung der Haut bei diabetischer Ulzera.

4. Erythropoietin (EPO) zur therapeutischen Anwendung bei der topischen Behandlung der Haut bei der Wundheilung nach Aufrauhung des Untergrundes im Rahmen eines Debridements und Applikation von EPO, wobei das EPO durch Calciumchlorid ausgelöste Polymerisation in ein Blutkoagel integriert und aus diesem langsam lokal abgeben wird, oder in Verbindung mit einem Fibrinkleber, einem Fliess oder einer mit EPO imprägnierten Tamponade verabreicht wird.

## Claims

1. Use of erythropoietin (EPO) for the preparation of a medicament for the topical treatment of the skin during wound healing after roughening of the substratum as part of debridement and application of EPO, where the EPO is integrated into a blood clot by calcium chloride-initiated polymerisation and is slowly released locally therefrom into the wound.

2. Use of EPO for the preparation of a medicament for the topical treatment of the skin during wound healing after roughening of the substratum as part of debridement and application of EPO, where the EPO is administered in combination with a fibrin adhesive, a nonwoven fabric or an EPO-impregnated tamponade.

3. Use according to Claim 1 or 2 for the topical treatment of the skin in the case of diabetic ulcers.

4. Erythropoietin (EPO) for therapeutic use in the topical treatment of the skin during wound healing after roughening of the substratum as part of debridement and application of EPO, where the EPO is integrated into a blood clot by calcium chloride-initiated polymerisation and is slowly released locally therefrom, or is administered in combination with a fibrin adhesive, a nonwoven fabric or an EPO-impregnated tamponade.

## Revendications

1. Utilisation d'érythropoïétine (EPO) pour la préparation d'un médicament destiné au traitement topique de la peau lors d'une guérison de plaie après avoir rendu le substratum rugueux dans le cadre d'un débridement, et application d'EPO, **caractérisée en ce que** l'EPO est intégrée dans un caillot sanguin par une polymérisation initiée par du chlorure de calcium et est lentement libérée localement dans la plaie à partir de celui-ci.

2. Utilisation d'EPO pour la préparation d'un médicament destiné au traitement topique de la peau lors d'une guérison de plaie après avoir rendu le substratum rugueux dans le cadre d'un débridement, et application d'EPO, **caractérisée en ce que** l'EPO est administrée en combinaison avec un adhésif à base de fibrine, un tissu non tissé ou une tamponnade imprégnée d'EPO.

3. Utilisation selon la revendication 1 ou 2, destinée au traitement topique de la peau dans le cas d'ulcères diabétiques.

4. Erythropoïétine (EPO) destinée à une utilisation thérapeutique dans le traitement topique de la peau lors d'une guérison de plaie après avoir rendu le substratum rugueux dans le cadre d'un débridement, et application d'EPO, **caractérisée en ce que** l'EPO est intégrée dans un caillot sanguin par une polymérisation initiée par du chlorure de calcium et est lentement libérée localement à partir de celui-ci, ou est administrée en combinaison avec un adhésif à base de fibrine, un tissu non tissé ou une tamponnade imprégnée d'EPO.
